(19) Europäisches Patentamt
European Patent Office
Office européen des brevets

(11) Publication number: **0 067 258**
**B1**

(12) # EUROPEAN PATENT SPECIFICATION

(45) Date of publication of patent specification: **17.04.85**

(51) Int. Cl.⁴: **C 07 C 49/623**, C 07 C 45/34, B 01 J 31/00

(21) Application number: **81302730.7**

(22) Date of filing: **17.06.81**

(54) **The preparation of cyclic ketones by catalytic oxidation of deltacarene (-3).**

(43) Date of publication of application:
**22.12.82 Bulletin 82/51**

(45) Publication of the grant of the patent:
**17.04.85 Bulletin 85/16**

(84) Designated Contracting States:
**CH DE FR IT LI NL**

(56) References cited:
**US-A-3 996 164**

(73) Proprietor: **INDIAN EXPLOSIVES LIMITED**
**ICI House 34 Chowringhee Road**
**Calcutta-700 071 West Bengal (IN)**

(73) Proprietor: **THE ALKALI AND CHEMICAL CORPORATION OF INDIA LIMITED**
**ICI House, 34 Chowringhee Road**
**Calcutta 700 071 West Bengal (IN)**

(73) Proprietor: **CHEMICALS AND FIBRES OF INDIA LIMITED**
**Crescent House 19 Walchand Hirachand Marg**
**Bombay-400 038 Maharashtra (IN)**

(72) Inventor: **Bhaduri, Sumit c/o Alcemie R.C.P.L.**
**Cafi Site, P.O. Box 155 Belapur Road**
**Thane-400 601 Maharashtra (IN)**
Inventor: **Mahandru, Madan Mohan c/o Alchemie R.C.P.L.**
**Cafi Site, P.O. Box 155 Belapur Road**
**Thane-400 601 Maharashtra (IN)**

(74) Representative: **Collier, Jeremy Austin Grey et al**
**J.A.Kemp & Co. 14, South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 067 258 B1

Courier Press, Leamington Spa, England.

**Description**

The present invention relates to the manufacture of ketonic derivatives of mono or sesqui-terpenic olefins by catalytic oxidation.

Of the many substrates which have been oxidised in the presence of transition metal complexes, one of the most extensively studied groups of organic compounds has been olefin hydrocarbons.

It is known, for example, from British Patent Specification No. 1256734 to oxidize oil of terpentine, which is essentially a mixture of α- and β-pinene, with molecular oxygen in the presence of a transition metal catalyst to produce a variety of oxygen-containing products. However, the oxidation of unsaturated compounds with molecular oxygen has previously been thought to be capable of giving only mono-ketonic products.

It has now surprisingly been found that the terpenic olefin $\Delta^3$-carene may be selectively oxidized with molecular oxygen to give a diketonic product. This opens up the possibility of further functionalisation of the parent olefin. The oxidized products can in most cases be converted into valuable chemicals.

The present invention provides a process for the preparation of 3,7,7-trimethylbicyclo[4.1.0]hept-3-en-2,5-dione which comprises reacting $\Delta^3$-carene with oxygen or an oxygen-containing gas in the presence of, as catalyst, a carboxylate salt or co-ordination or organometallic complex of V, Cr, Mn, Fe, Rh, Co, Ru, Ir, Ni, Pd, Pt or Cu.

The corresponding mono-ketone may be formed at the same time.

The following diagram illustrates this conversion:

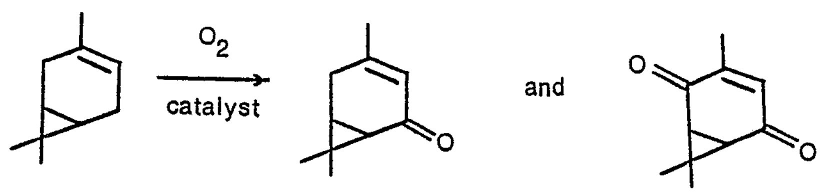


$\Delta^3$ –carene

and

(1)

(2)

Compound (1) is 3,7,7-trimethylbicyclo[4.1.0]hept-3-en-5-one and compound (2) is 3,7,7-trimethylbicyclo-[4.1.0]hept-3-en-2,5-dione.

Although the conversion into a mixture of mono- and diketonic derivatives can be effected by complexes of all the metals listed above, for high conversions and selectivities, the preferred metals are Mn(II) or Mn(III), Co(II) or Co(III), Cu(I) or Cu(II) and Fe(II) or Fe(III). The reactions presumably proceed through the well-known Haber-Weiss mechanism of radical auto-oxidation.

It is known that under the conditions used for similar oxidations of olefins the original ligand environment of the metal ion does not remain unchanged. The effectiveness of the catalyst partly depends on its solubility and, therefore, any soluble compound of the preferred metals should be able to initiate and propagate the radical auto-oxidation in varying degree. Apart from the carboxylates such as stearates, oleates, acelates and benzoates of Co(II or III), Mn(II or III) and Cu(I or II) which have been widely used for the other olefin oxidation reactions, the catalysts can be any one or more of the compounds represented by the formulae A, B, C and D as depicted below:

$$M_m(L_2)_nX_p(O)_q$$

Formula A

where

$L_2$ = 2,2′ bipyridyl or substituted 2,2′ bipyridyl or substituted or unsubstituted 1,10-orthophenanthroline; and

a) M = Cu, m = 2, n = 2, X = 1, Br or Cl and p= 2, q = 0; or

b) M = Cu, m = 1, n = 1, X = $NO_2$, p = 2, q = 0; or

c) M = Mo, m = 1, n = 1, X = Br, p = 2, q = 2

$$M(O)_m(R_1CO)CH(COR_2)_n$$

Formula B

where,

a) M = V, m = 1, n = 2, $R_1$ and $R_2$ which may be the same or different, represent $CH_3$, $C_2H_5$, n-$C_3H_7$, $CF_3$, $C_6H_5$, $C_6H_5CH_2$, or

b) M = Mo, m = 2, n = 2, $R_1$ and $R_2$ as shown in (a), or

c) M = Co, m = 0, n = 2, $R_1$ and $R_2$ as shown in (a), or

d) M = Co, m = 0, n = 3, $R_1$ and $R_2$ as shown in (a), or
e) M = Mn, m = 0, n = 2 as shown in (a), or
f) M = Cu, m = 0, n = 2 as shown in (a), or
g) M = Rh, m = 0, n = 3 as shown in (a)
M = Fe, m = 0, n = 3 as shown in (a)

$$ML_nX_p$$

Formula C

where,
a) M = Rh, L = $PPh_3$, n = 3, X = Cl, p = 1, or
b) M = Rh, L = $PPh_3$, n = 3, X = Cl, p = 2

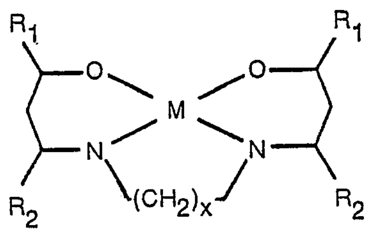

Formula D

where,
M = Co, x = 2 or 3, $R_1$ and $R_2$ which may be the same or different and represent $CH_3$, $C_2H_5$, $C_3H_7$, $CF_3$, Ph, $PhCH_2$.

The oxidation reaction can be effected at a temperature from 0°C to 250°C and preferably at ambient room temperature up to 100°C. Both air or pure oxygen can be used as the oxidising agent. The pressures employed are in the range of atmospheric (101 kPa) to 1000 psi (6895 kPa). Optimisation of temperature and pressure is required depending on the desired ratio of the mono- to diketonic derivatives.

The residence time of the reaction can be followed by taking periodic gas-chromatographic samples of the product mixture. The products are first isolated by chromatographic techniques and structures established by the usual spectroscopic methods. These are then used for rapid gas-chromatographic analysis. The reaction time varies with the desired ratio of mono- to diketonic derivatives, the pressure employed, the temperatures used and the like considerations.

In the practice of these processes undiluted $\Delta^3$-carene is preferably used but it can also be diluted with solvents. The solvents employable include by way of example carbon tetrachloride, benzene, n-octane, decalin and ethyl acetate.

The following Examples are given by way of illustration of the invention.

Example 1

$\Delta^3$-carene (20 parts) was contacted with bis (pentane-2,4-dionato) cobalt (II) (0.2 part) under 50 psi (345 kPa) of oxygen for 60 minutes. The reaction mixture was vigorously stirred. The products were analysed by gas liquid chromatography showing 75% conversion of $\Delta^3$-carene. The converted material consisted of the 5-ketocarene (60%), 2,5-diketo-carene (10%) and other products (5%).

Example 2

$\Delta^3$-carene (20 parts) was contacted with cobalt (II) stearate (0.5 part) under 250 psi (1724 kPa) of oxygen at 80°C for 24 hours. The reaction mixture was vigorously stirred. The products were analysed by gas-liquid chromatography showing 95% conversion of $\Delta^3$-carene. The converted material consisted of the 5-ketocarene (30%), 2,5-diketo-carene (50%) and other products (15%).

**Claims**

1. A process for the preparation of 3,7,7-trimethylbicyclo[4.1.0]hept-3-en-2,5-dione which comprises reacting $\Delta^3$-carene with oxygen or an oxygen-containing gas in the presence of, as catalyst, a carboxylate salt or a co-ordination or organometallic complex of V, Cr, Mn, Fe, Rh, Co, Ru, Ir, Ni, Pd, Pt, or Cu.

2. A process as claimed in claim 1, in which the oxygen-containing gas is air.

3. A process as claimed in claim 1 or 2, wherein the reaction is conducted at from 0°C to 250°C, preferably from ambient room temperature to 100°C.

4. A process as claimed in any one of claims 1 to 3, wherein the reaction is conducted at a pressure of from 1 atmosphere (101 kPa) to 1000 psi (6895 kPa), preferably 50—400 psi (345—2758 kPa).

5. A process as claimed in any one of claims 1 to 4, in which the reaction is conducted in the presence of an inert liquid diluent.

6. A process as claimed in any of claims 1 to 5, wherein the catalyst is a carboxylate salt, co-ordination or organo-metallic complex of manganese, cobalt, copper or iron.

7. A process as claimed in any one of claims 1 to 5, wherein the catalyst has the formula $M_m(L_2)_nX_p(O)_q$ where $L_2$ is 2,2'-bipyridyl or substituted 2,2'-bipyridyl or substituted or unsubstituted 1,10-orthophenanthroline, and either

(a) M is Cu, m = 2, X is I, Br or Cl, p = 2, q = 0; or
(b) M is Cu, m = 1, n = 1, X is $NO_2$, p = 2 and q = 0; or
(c) M is Mo, m = 1, n = 1, X = Br, p = 2 and q = 2.

8. A process as claimed in any one of claims 1 to 5, wherein the catalyst has the formula $M(O)_m(R_1CO)CH(COR_2)_n$ where $R_1$ and $R_2$ which may be the same or different represent methyl, ethyl, n-propyl, trifluoromethyl, phenyl or benzyl, and either

(a) M is V, m = 1, n = 2; or
(b) M is Mo, m = 2, n = 2; or
(c) M is Co, m = 0, n = 2; or
(d) M is Co, m = 0, n = 3, or
(e) M is Mn, m = 0, n = 2; or
(f) M is Cu, m = 0, n = 2; or
(g) M is Rh, m = 0, n = 3; or
(h) M is Fe, m = 0, n = 3.

9. A process as claimed in any one of claims 1 to 5, wherein the catalyst has the formula $ML_nX_p$ where M is Rh, L is triphenylphosphine, n = 3, X = Cl and p = 1 or 2.

10. A process as claimed in any of claims 1 to 5, wherein the catalyst has the formula

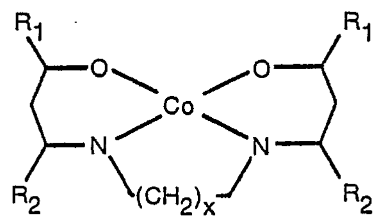

where x = 2 or 3 and $R_1$ and $R_2$ which may be the same or different represent methyl, ethyl, propyl, trifluoromethyl, phenyl or benzyl.

11. A process as claimed in any of claims 1 to 10, wherein $\Delta^3$-carene is converted into 3,7,7-trimethylbicyclo[4.1.0]hept-3-en-5-one and 3,7,7-trimethylbicyclo(4.1.0)hept-3-en-2,5-dione.

## Patentansprüche

1. Verfahren zur Herstellung von 3,7,7-Trimethylbicyclo[4.1.0]hept-3-en-2,5-dion, dadurch gekennzeichnet, daß $\Delta^3$-Caren mit Sauerstoff oder einem Sauerstoff enthaltenden Gas in Gegenwart eines als Katalysator diene den Carboxylatsalzes oder Koordinations- oder organometallischen Komplexes von V, Cr, Mn, Fe, Rh, Co, Ru, Ir, Ni, Pd, Pt oder Cu umgesetzt wird.

2. Verfahren nach Anspruch 1, dadurch gekennzeichnet, daß das Sauerstoff enthaltende Gas Luft ist.

3. Verfahren nach Anspruch 1 oder 2, dadurch gekennzeichnet, daß die Reaktion bei 0°C bis 250°C, vorzugsweise bei Raumtemperatur bis 100°C ausgeführt wird.

4. Verfahren nach einem der Ansprüche 1 bis 3, dadurch gekennzeichnet, daß die Reaktion bei einem Druck von 1 Atmosphäre (101 kPa) bis 1000 psi (6895 kPa), vorzugsweise bei 50—400 psi (345—2758 kPa) ausgeführt wird.

5. Verfahren nach einem der Ansprüche 1 bis 4, dadurch gekennzeichnet, daß die Reaktion in Gegenwart eines inerten flüssigen Verdünnungsmittels ausgeführt wird.

6. Verfahren nach einem der Ansprüche 1 bis 5, dadurch gekennzeichnet, daß der Katalysator ein Carboxylatsalz, ein Koordinations- oder ein organometallischer Komplex des Mangans, Kobalts, Kupfers oder Eisens ist.

7. Verfahren nach einem der Ansprüche 1 bis 5, dadurch gekennzeichnet, daß der Katalysator die Formel $M_m(L_2)_nX_p(O)_q$ hat, in der $L_2$ 2,2'-Bipyridyl oder substituiertes 2,2'-Bipyridyl oder unsubstituiertes oder substituiertes 1,10-Orthophenanthrolin ist und entweder

(a) M Cu ist, m = 2, X I, Br oder Cl ist, p = 2, q = 0; oder
(b) M Cu ist, m = 1, n = 1, X $NO_2$ ist, p = 2 und q = 0; oder
(c) M Mo ist, m = 1, n = 1, X Br ist, p = 2 und q = 2.

8. Verfahren nach einem der Ansprüche 1 bis 5, dadurch gekennzeichnet, daß der Katalysator die Formel $M(O)_m(R_1CO)CH(COR_2)_n$ hat, in der $R_1$ und $R_2$, die gleich oder verschieden sein können, Methyl, Ethyl, n-Propyl, Trifluormethyl, Phenyl oder Benzyl bedeuten und entweder

(a) M V ist, m = 1, n = 2; oder
(b) M Mo ist, m = 2, n = 2; oder
(c) M Co ist, m = 0, n = 2; oder
(d) M Co ist, m = 0, n = 3; oder
(e) M Mn ist, m = 0, n = 2; oder
(f) M Cu ist, m = 0, n = 2; oder
(g) M Rh ist, m = 0, n = 3; oder
(h) M Fe ist, m = 0, n = 3.

9. Verfahren nach einem der Ansprüche 1 bis 5, dadurch gekennzeichnet, daß der Katalysator die Formel $ML_nX_p$ hat, in der M Rh ist, L Triphenylphosphin ist, n = 3, X Cl ist und p = 1 oder 2.

10. Verfahren nach einem der Ansprüche 1 bis 5, dadurch gekennzeichnet, daß der Katalysator die Formel

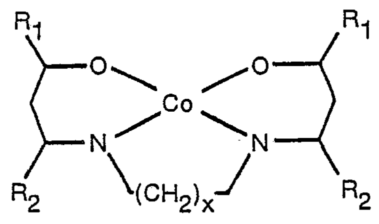

hat, in der x = 2 oder 3 und $R_1$ und $R_2$, die gleich oder verschieden sein können, Methyl, Ethyl, Propyl, Trifluormethyl, Phenyl oder Benzyl bedeuten.

11. Verfahren nach einem der Ansprüche 1 bis 10, dadurch gekennzeichnet, daß $\Delta^3$-Caren in 3,7,7-Trimethylbicyclo[4.1.0]hept-3-en-5-on und 3,7,7-Trimethylbicyclo(4.1.0)hept-3-en-2,5-dion umgewantelt wird.

**Revendications**

1. Procédé pour la préparation du triméthyl-3,7,7-bicyclo(4,1,0)heptène-3-dione-2,5, dans lequel on fait réagir du $\Delta^3$-carène avec de l'oxygène ou un gaz contenant de l'oxygène en présence, comme catalyseur, d'un carboxylate ou d'un complexe de coordination ou organométallique de vanadium chrome, manganèse, fer, rhodium, cobalt, ruthénium, iridium, niobium, palladium, platine ou cuivre.

2. Procédé selon la revendication 1, dans lequel le gaz contenant de l'oxygène est l'air.

3. Procédé selon la revendication 1 ou la revendication 2, dans lequel la réaction est conduite à une température comprise entre 0°C et 250°C, de préférence entre la température ambiante et 100°C.

4. Procédé selon l'une quelconque des revendications 1 à 3, dans lequel la réaction est conduite à une pression comprise entre 101 kPa et 6895 kPa, et de préférence entre 345 kPa et 2758 kPa.

5. Procédé selon l'une quelconque des revendications 1 à 4, dans lequel la réaction est conduite en présence d'un diluant liquide inerte.

6. Procédé selon l'une quelconque des revendications 1 à 5, dans lequel le catalyseur est un carboxylate, un complexe de coordination ou un complexe organométallique de manganèse, de cobalt, de cuivre ou de fer.

7. Procédé selon l'une quelconque des revendications 1 à 5, dans lequel le catalyseur a la formule $M_m(L_2)_nX_p(O)_q$, dans laquelle $L_2$ est le bipyridyl-2,2' ou le bipyridyl-2,2' substitué ou l'orthophénanthroline, substituée ou non substituée, et dans laquelle:
(a) M est Cu, m = 2, X est I, Br ou Cl, p = 2, q = 0; ou
(b) M est Cu, m = 1, n = 1, X est $NO_2$, p = 2 et q = 0; ou
(c) M est Mo, m = 1, n = 1, X = Br, p = 2 et q = 2.

8. Procédé selon l'une quelconque des revendications 1 à 5, dans lequel le catalyseur a la formule $M(O)_m(R_1CO)CH(COR_2)_n$ où $R_1$ et $R_2$, qui peuvent être identiques ou différents, représentent le méthyle, l'éthyle, le n-propyle, le trifluorométhyle, le phényle ou le benzyle, et dans laquelle
(a) M est V, m = 1, n = 2; ou
(b) M est Mo, m = 2, n = 2; ou
(c) M est Co, m = 0, n = 2; ou
(d) M est Co, m = 0, n = 3; ou
(e) M est Mn, m = 0, n = 2; ou
(f) M est Cu, m = 0, n = 2; ou
(g) M est Rh, m = 0, n = 3; ou
(h) M est Fe, m = 0, n = 3.

9. Procédé selon l'une quelconque des revendications 1 à 5, dans lequel le catalyseur a la formule $ML_nX_p$, où M est Rh, L est la triphénylphosphine, n = 3, X = Cl et p = 1 ou 2.

10. Procédé selon l'une quelconque des revendications 1 à 5, dans lequel le catalyseur a la formule:

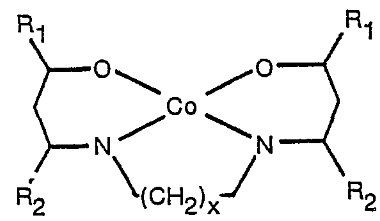

où x = 2 ou 3 et $R_1$ et $R_2$, qui peuvent être identiques ou différents, représentent le méthyle, l'éthyle, le propyle, le trifluorométhyle, le phényle ou le benzyle.

11. Procédé selon l'une quelconque des revendications 1 à 10, dans lequel on transforme le $\Delta^3$-carène en triméthyl-3,7,7-bicyclo(4.1.0)heptène-3-one-5 et en triméthyl-3,7,7-bicyclo(4.1.0)heptène-3-dione-2,5.